# EUROPEAN PATENT SPECIFICATION

(11) **EP 1 324 749 B1**
(45) Date of publication and mention of the grant of the patent: **15.03.2006**
(21) Application number: 01979338.9
(22) Date of filing: 01.10.2001
(51) Int. Cl.: A61K 9/00

(54) **MEDICINAL AEROSOL FORMULATIONS**
MEDIZINISCHE AEROSOLZUSAMMENSETZUNG
FORMULATIONS MEDICINALES EN AEROSOL

(30) Priority: 09.10.2000 GB 0024711; 18.09.2001 GB 0122512
(43) Date of publication of application: 09.07.2003
(73) Proprietor: 3M Innovative Properties Company, St. Paul, MN 55133-3427 (US)
(72) Inventor: JINKS, Philip A., Loughborough, Leicestershire LE11 3JS (GB); MCKENZIE, Lesley, Mapperley, Nottinghamshire NG3 6FH (GB); LISTER, James T., Nottingham, Nottinghamshire NG11 7AN (GB)
(74) Representative: Vossius & Partner
(86) International application number: PCT/US2001/030575
(87) International publication number: WO 2002/030394

(56) References cited:
- WO-A-96/25918
- US-A- 5 254 330
- US-A- 5 747 001

## Description

This invention relates to medicinal aerosol formulations and in particular to suspension aerosol formulations containing drug particles and a nanoparticulate auxiliary powder suitable for administration to the respiratory tract.

Medicinal aerosol formulations in pressurised containers have been available for over forty years. For most of this time, chlorofluorocarbons have been used as the propellants. Drugs have been formulated either as solutions or as suspensions, depending on their solubility properties and other factors. Following environmental concerns over their use, other propellants have been introduced, and this has presented a challenge to reformulate or to introduce new drugs, as well as an opportunity to provide improved pharmaceutical performance.

Two propellants that have emerged as favourites are 1,1,1,2-tetrafiuoroethane (HFA 134a) and 1,1,1,2,3,3,3-heptafluoropropane (HFA 227). These have distinctly different solvent properties to the chlorofluorocarbons, and this has had a bearing on the properties of formulations.

When formulating, suspensions, micronised- drug is dispersed in a propellant system with other ingredients added as appropriate for maintaining the stability of the formulation. One aspect of stability is the homogeneity of the dispersed drug, which can sediment (settle) or cream (float) depending on the density difference between drug and propellant, or it can flocculate, which requires some degree of agitation to deflocculate it. Such challenges are presented when formulating suspensions of any drug, but are particularly important when high potency drugs, such as Formoterol, Fluticasone Propionate, Salmeterol, Procaterol and Ipratropium and salts thereof are formulated.

When more potent drugs are formulated as suspensions, the concentration of drug required is lower than for less potent drugs. Sedimenting, creaming or flocculating drug leads to greater inhomogeneity of the contents that in tum may lead to delivery of incorrect doses when the formulation is dispensed from the metering valve.

Formulations of more potent drugs have been prepared as suspensions in hydrofluoroalkanes, such as those disclosed in WO 97/47286 (herein incorporated by reference) but it is still possible to make further improvements to the homogeneity of such formulations.

Various suggestions have been made to improve the quality of pharmaceutical aerosol formulations.

WO 00/27363 and US-A- 5747001 disclose aerosol formulations comprising droplets of an aqueous dispersion of nanoparticles comprising insoluble drug having a surface modifier on the surface thereof. The nanoparticles generally have an effective average particle size of less than about 1000nm.

EP-A-0768114 discloses a method for homogenizing and micronising aerosol formulations in a closed apparatus under elevated pressure. The apparatus includes a closed loop containing a reaction vessel and homogenizer. The homogenizer includes an interaction chamber and an intensfier pump; the interaction chamber includes a stream splitter for separating a stream of aerosol formulation into two streams and an impaction chamber for combining the streams. Aerosol formulations comprising ipratropium bromide and albuterol sulphate and optionally surfactant in CFC propellants and HFA 227 are disclosed with particle sizes of 5 to 10 micron.

US-A-5711934 discloses a process for preparing aerosol formulations by milling the formulation in aerosol propellant at a temperature between -80° C and 10° C. Particle sizes of less than 10 micron are obtained.

EP-A-0726088 discloses a process for preparing aerosol compositions in which the composition is maintained under constant recirculation under high pressure conditions and forced through plates with a plurality of micro-apertures until obtaining a uniform dispersion. No particle sizes are disclosed.

WO 00/25746 disclose a process for the preparation of suspensions of drug particles for inhalation delivery which includes the step of homogenizing the formulation in a turboemulsifier provided with a high-potency turbine, optionally followed by a treatment in a high-pressure homogenizer. The Examples demonstrate the preparation of an aqueous suspension of beclomethasone dipropionate.

US-A-6086376 discloses aerosol formulations containing stabilized particles of drug having a mean size range of 0.1 to 10 micron coated with a membrane-forming, amphiphatic lipid and dispersed in HFA 134a or HFA 227 propellant.

US-A-5858410 discloses a drug carrier comprising particles of at least one pure active compound which is insoluble, only sparingly soluble or moderately soluble in water, aqueous media and/or organic solvents, wherein the active ingredient is solid at room temperature and has an average diameter, determined by photon correlation spectroscopy of 10nm to 1 000nm, the proportion of particles larger than 5 micron in the total population being less than 0.1 % (number distribution). It is suggested that for metered aerosols spraying a powder the drug carriers in the nanometre range are sprayed onto carrier particles, such as lactose, in the micrometre range. The lactose dissolves in the lung releasing the drug carriers.

### Summary of the Invention

In one aspect the present invention relates to the use of a particulate bulking agent having a mass median diameter of less than one micron in pharmaceutical aerosol formulations comprising drug in a suspension of particles in a propellant

In accordance with a second aspect of the Invention there is provided a pharmaceutical aerosol formulation comprising particles of drug dispersed in a propellant and a bulking agent having a mass median diameter of less than one micron.

It has been found that improved suspension aerosol formulations of drug can be prepared by introducing a bulking agent having a mass median diameter of less than one micron. The bulking agent improves the stability of the suspension of drug particles which may be micronised drug particles or other drug particles having a mass median diameter equal to or greater than 1 micron (more particularly from 1 to 10 micron, even more particularly from 1 to 5 micron) or smaller particles having a mass median diameter of less than one micron. It is not necessary for the surface of the bulking agent or the drug to be coated with a surface modifier to achieve improved stability.

Mass median diameter (which is equivalent to volume median diameter) can be determined using any conventional particle size measurement method known to those skilled in the art. Suitable methods include for example laser diffraction, photon correlation spectroscopy (e.g. using a spectrometer available under the trade designation Brookhaven PCS from Brookhaven Inc.), spinning disc centrifuging (using an instrument available under the trade designation CPS Disc Centrifuge from Chemical Process Specialists Inc.), and scanning electron microscopy (SEM). Mass median diameter is preferably determined by laser diffraction, more particularly laser diffraction using an analyser available under the trade designation Malvern Mastersizer 2000 laser light diffraction particle size analyser from Malvern Instruments Ltd.

Although potent drugs (i.e. a drug having a potency such that concentrations of the drug in a formulation of less than about 0.1 % w/w are therapeutically effective), such as Formoterol, Fluticasone Propionate, Salmeterol, Procaterol, Ipratropium and salts thereof benefit particularly from this invention, it is applicable to any medicament to be prepared as a suspension formulation. Other drugs which may be used in aerosol formulations are well known and are referred to in the above literature references. Non-limiting examples of other suitable drugs include antiallergics, analgesics, bronchodilators, antihistamines, therapeutic proteins and peptides, antitussives, anginal preparations, antibiotics, anti-inflammatory preparations, hormones, or sulfonamides, such as, for example, a vasoconstrictive amine, an enzyme, an alkaloid or a steroid, and combinations of these specific examples or drugs which may be employed are: isoproterenol [alpha-(isopropylaminomethyl) protocatechuyl alcohol], phenylephrine, phenylpropanolamine, glucagon, adrenochrome, trypsin, epinephrine, ephedrine, narcotine, codeine, atropine, heparin, morphine, dihydromorphinone, ergotamine, scopolamine, methapyrilene, cyanocobalamin, terbutaline, rimiterol, salbutamol, isoprenaline, fenoterol, oxitropium bromide, reproterol, budesonide, flunisolide, ciclesonide, triamcinolone acetonide, mometasone furoate, colchicine, pirbuterol, beclomethasone dipropionate, orciprenaline, fentanyl, and diamorphine. Others are antibiotics, such as neomycin, streptomycin, penicillin, procaine penicillin, tetracycline, chlorotetracycline and hydroxytetracycline; adrenocorticotropic hormone and adrenocortical hormones, such as cortisone, hydrocortisone, hydrocortisone acetate and prednisolone; antiallergy compounds such as cromolyn sodium, nedocromil, protein and peptide molecules such as insulin, pentamidine, calcitonin, amiloride, interferon, LHRH analogues, DNAase, heparin, etc. The drugs exemplified above may be used as either the free base or as one or more salts known to the art. Vaccines may also benefit from this approach.

Preferred bulking agents include lactose, DL-alanine, ascorbic acid, glucose, sucrose D(+)trehalose as well as their various hydrates, anomers and/or enantiomers. Lactose (including its various forms, such as α-lactose monohydrate and β-lactose) is more preferred as a bulking agent due to e.g. processing considerations. Other suitable bulking agents include other saccharides e.g. D-galactose, maltose, D(+)raffinose pentahydrate, sodium saccharin, polysaccharides e.g. starches, modified celluloses, dextrins or dextrans, other amino acids e.g. glycine, salts e.g. sodium chloride, calcium carbonate, sodium tartrate, calcium lactate, or other organic compounds e.g. urea or propyliodone.

The weight ratio of drug to bulking agent is generally in the range 1:0.1 to 1:100. Preferably the weight ratio is in the range 1:5 to 1:40, although lower ratios of drug to bulking agent will be required for less potent drugs.

The concentration of drug depends largely on its potency. This invention is particularly applicable to drugs formulated at a concentration of less than 0.1 % w/w.

For drugs formulated at a concentration of less than 0.1 % w/w, a weight ratio of drug to bulking agent in the range 1:10 to 1:30 has been found particularly suitable, and a weight ratio of about 1:20 most suitable. For drugs formulated at a concentration equal to or greater than 0.1 % w/w, a weight ratio of drug to bulking agent in the range 1:0.1 to 1:10 has been found particularly suitable, a weight ratio in the range 1:0.5 to 1:5 even more suitable, and a weight ratio in the range 1:1 to 1:2 most suitable.

In other preferred embodiments according to the present invention, bulking agents include an active ingredient. In particular for pharmaceutical aerosol formulations comprising two (or more) drugs, one drug, besides acting as an active ingredient, can be desirably applied in the formulation as a particulate bulking agent in accordance with the present invention. Although the concentration of each drug in such a formulation depends largely on its potency, the weight ratios of drug to bulking agent as described above are applicable, wherein the drug applied as the bulking agent is understood under "bulking agent" and the other drug (or drugs) being bulked is understood under "drug". This approach is particularly desirable for improving stability of suspension formulations wherein the drug (or drugs) being bulked has a mass median diameter equal to or greater than 1 micron (more preferably from 1 to 10 micron, most preferably from 1 to 5 micron). This approach is also particularly advantageous for pharmaceutical aerosol formulations comprising a potent drug and a second, preferably a less potent, drug, wherein the second drug is applied as a particulate bulking agent in the formulation. Preferred drug combinations include: a potent bronchodilator, such as Formoterol, Salmeterol, Procaterol, lpratropium bromide and salts thereof, in combination with an anti-inflammatory, such as budesonide, flunisolide, ciclesonide, triamcinolone acetonide, mometasone furoate and beclomethasone dipropionate, as the bulking agent; and a potent anti-inflammatory, such as Fluticasone Propionate, in combination with a bronchodilator, such as isoproterenol, terbutaline, rimiterol, salbutamol, reproterol, pirbuterol, orciprenaline and salts thereof, as the bulking agent.

The bulking agent may be reduced to the required particle size by any convenient method, e.g. grinding, air-jet milling etc. Preferably the bulking agent is reduced to nanoparticle size in a high pressure homogenizer, such as the commercially available Avestin Emulsiflex homogenizers and the Microfluidics Microfluidizer homogenizers. Surprisingly in the processing with high pressure homogenizers, certain bulking agents can be reduced to the desired particle size using lower pressures than that applied for other bulking agents. For example, it has been found that lactose, more specifically
α-lactose monohydrate, can be effectively reduced to the desired particle size using pressures between about 10,000 and about 21,000 psi, while for effective particle size reduction of alanine or sucrose higher pressures of about 25,000 psi were applied. The mass median diameter of the bulking agent can advantageously be as low as 300 nanometers, more desirably as low as 250 nanometers and most desirably the mass median diameter is in the range of 100 to 250 nanometers.

The bulking agent may be prepared in a slurrying aid which is a low volatility solvent such as ethanol. It may be prepared in a slurrying aid which is a component of the final aerosol formulation, or it may be prepared in a solvent that is subsequently removed or exchanged with a component of the formulation by some process such as centrifugation and decanting, dialysis, evaporation etc. Volatile ingredients of the formulation may be used as the slurrying aid, such as propellants, provided the homogenizer and any associated pipework or product reservoirs are built to withstand the propellant pressure.

It is particularly convenient to use a slurrying aid in the high pressure homogenizer which is a low volatility component of the aerosol formulation and after particle size reduction has been achieved the slurry may be adjusted if necessary, e.g. concentrated by centrifugation, decanting etc. Whilst it has been found that slurries with excessively high powder loadings may be difficult to process due to their rheological properties, it is generally advantageous to process slurries with powder loading concentrations which approach this processing limit in order to achieve the desired particle size distribution in the shortest processing time. Thus, the weight ratio of liquid:solid is generally in the range 5:1 to 100:1, preferably 5:1 to 20:1, and most preferably about 10:1.

According to a further aspect of the invention there is provided a process for the preparation of an aerosol formulation comprising a suspension of particles in propellant which process comprises forming a slurry of bulking agent with a component of the aerosol formulation, subjecting the slurry to high pressure homogenization to reduce the particle size of the bulking agent, and thereafter mixing the resulting slurry with other components of the aerosol formulation.

The aerosol formulations of the invention may contain ethanol, generally in an amount in the range 0.1 to 5% by weight, preferably from about 0.5 to about 3% by weight, more preferably from about 1 to about 2% by weight.

The aerosol formulations of the invention may optionally contain surfactant. Suitable surfactants are well known in the art and include sorbitan trioleate , oleic acid and lecithin. Surfactants, such as oligolactic acid derivatives disclosed in WO94/21228 and WO94/21229, and other surfactants disclosed in the literature may be used.

The invention will be illustrated by the following Examples.

### Example 1

α-lactose monohydrate supplied under the trade designation Pharmatose 325M by DMV International Pharma was micronised by fluid energy milling in a single pass (referred to here and in the following as "micronised lactose monohydrate"). Micronised lactose monohydrate (15g) was then dispersed in anhydrous ethanol (500g). This dispersion was added to the product reservoir of an Avestin Emulsiflex C50 homogeniser. The pressure was stepped up according to the following protocol:

| Length of time (mins) | Pressure (psi) |
|---|---|
| 5 | 0 |
| 5 | 5,000 |
| 5 | 10,000 |
| 10 | 15,000 |
| 5 | 15,000 |
| 10 | 18,000 |

Microscopic analysis of the product after 40 minutes showed it to be considerably less than 1 micron. The product was also examined by Scanning Electron Microscopy (SEM), and the particle size estimated to be 100 nm. Quantities of dispersions sampled after 5 minutes and after 40 minutes of the above protocol (1.5g) were added to a 20ml clear pressure resisting plastic vials and non-metering valves then crimped in place. HFA 134a (20g) was then injected through each valve. The suspension formulation from the 40 minute sample was seen to have a markedly lower sedimentation rate and a larger sediment bulk on standing compared to the 5 minute sample, both features of which are advantageous for a bulking agent in metered dose inhaler suspension formulations. Hence a technique has been found to provide a stable suspension of an excipient (lactose) which in the particle size range typically used for inhalation (2 to 5 microns) would have too rapid a sedimentation rate to be adequately stable but when further reduced in size to 300 nanometres provides a stable dispersion. The technique is likely to be applicable to a wide range of other compounds which by the degree of their density difference in comparison with HFA propellants, would in the size range typically used in inhaler suspension formulations, form poorly stable dispersions.

### Example 2

A slurry of micronised lactose monohydrate (15g) in anhydrous ethanol (500g) was processed according to Example 1.

The slurry of lactose was centrifuged for 5 minutes at 5000 rpm using 10g per tube, to concentrate it. After decanting off excess ethanol, a lactose to ethanol ratio of 1:4 was achieved. This was added as a thick paste to the inner walls of PET vials into which micronised Formoterol fumarate had been added, then charged with propellant to prepare formulations as follows:

| | mg/ml | g/unit | mg/ml | g/unit | mg/ml | g/unit |
|---|---|---|---|---|---|---|
| Formoterol fumarate | 0.132 | 0.0020 | 0.132 | 0.0017 | 0.132 | 0.0018 |
| Lactose monohydrate | 2.640 | 0.0390 | 2.640 | 0.0340 | 2.640 | 0.0357 |
| Ethanol | 12.180 | 0.1800 | 13.960 | 0.1800 | 13.317 | 0.1800 |
| HFA 134a | 1203.048 | 17.7790 | 0.000 | 0.0000 | 493.337 | 6.6684 |
| HFA 227 | 0.000 | 0.0000 | 1379.268 | 17.7843 | 822.228 | 11.1141 |

Each formulation was subjected to ultrasonic agitation in a water bath for one minute to ensure complete dispersion. The physical appearance was assessed visually and by using an optical measuring technique such as that described in the Proceedings of Drug Delivery to the Lung VI p.10-13 (December 1995) printed by the Aerosol Society.

The formulations were compared with three formulations of the same composition, in which the lactose monohydrate had been air-jet milled to a micronised size range similar to that of the drug.

All of the formulations with lactose processed according to example 1 sedimented more slowly than all of those with air-jet milled lactose, and they were therefore more stable in this respect.

### Example 3

Further formulations were prepared in a similar manner to Example 2 using nanoparticulate lactose and micronised lactose but without drug as follows:

| | mg/ml | g/unit | mg/ml | g/unit | mg/ml | g/unit |
|---|---|---|---|---|---|---|
| Lactose monohydrate | 2.772 | 0.0410 | 2.772 | 0.0357 | 2.772 | 0.0375 |
| Ethanol | 12.180 | 0.1800 | 13.960 | 0.1800 | 13.317 | 0.1800 |
| HFA 134a | 1203.048 | 17.7790 | 0.000 | 0.0000 | 493.337 | 6.6684 |
| HFA 227 | 0.000 | 0.0000 | 1379.268 | 17.7843 | 822.228 | 11.1141 |

Similar results were obtained using the optical measuring technique to those obtained for the formulations of Example 2, samples containing nanoparticulate lactose sedimented more slowly than all those samples containing air-jet milled micronised lactose.

### Example 4

Further formulations were made on a scale of about 20 inhalers as follows:

| | mg/ml | g/unit | mg/ml | g/unit |
|---|---|---|---|---|
| Formoterol | 0.1320 | 0.0010 | 0.1320 | 0.0010 |
| Lactose monohydrate | 0.5280 | 0.0040 | 2.6400 | 0.0198 |
| Ethanol | 1.9975 | 0.0150 | 13.3165 | 0.1000 |
| HFA 134a | 493.3370 | 3.7047 | 493.337 | 3.7047 |
| HFA227 | 822.2284 | 6.1745 | 822.228 | 6.1745 |

The formulations exhibited good visual stability.

### Example 5

Further formulations of Formoterol Fumarate were prepared on a small manufacturing scale (300 inhalers), in which a thick slurry of nanosized lactose prepared as in Example 2 but with a weight ratio of lactose : ethanol of 1:2.

The slurry was added to a stainless steel vessel. Oleic acid with any necessary additional ethanol was added. The mixture was homogenised for 5 minutes.

Propellants were added to a batching vessel. The slurry was added to the propellants. Some additional propellant was used to rinse the stainless steel vessel. Drug was then added to the batching vessel, and dispersed using a high shear mixer at 8000 rpm for one minute. The formulation was dispensed into canisters by cold-filling, then valves were crimped on.

| | mg/ml | g/unit | mg/ml | g/unit | mg/ml | g/unit |
|---|---|---|---|---|---|---|
| Formoterol fumarate | 0.1320 | 0.0010 | 0.1320 | 0.0010 | 0.1320 | 0.0010 |
| Lactose monohydrate | 2.6400 | 0.0218 | 2.6400 | 0.0190 | 2.6400 | 0.0200 |
| Oleic Acid | 0.0606 | 0.0005 | 0.0695 | 0.0005 | 0.0661 | 0.0005 |
| Ethanol | 24.2285 | 0.2000 | 27.798 | 0.2000 | 26.4595 | 0.2000 |
| HFA 134a | 1184.3653 | 9.7766 | 0.0000 | 0.0000 | 485.1290 | 3.6744 |
| HFA 227 | 0.0000 | 0.0000 | 1359.2644 | 9.7796 | 808.5483 | 6.1241 |

Further formulations without lactose were made on a small manufacturing scale for comparison, as follows:

| | mg/ml | g/unit | mg/ml | g/unit | mg/ml | g/unit |
|---|---|---|---|---|---|---|
| Formoterol fumarate | 0.1320 | 0.0011 | 0.132 | 0.0010 | 0.132 | 0.0010 |
| Oleic Acid | 0.0606 | 0.0005 | 0.0695 | 0.0005 | 0.0661 | 0.0005 |
| Ethanol | 24.2285 | 0.2000 | 27.7981 | 0.200 | 26.4595 | 0.2000 |
| HFA 134a | 1187.049 | 9.7984 | 0.0000 | 0.0000 | 486.119 | 3.6744 |
| HFA 227 | 0.0000 | 0.0000 | 1361.9044 | 9.7986 | 810.1983 | 6.1241 |

Initial medication delivery data for the inhalers manufactured on a small scale showed that those formulations with nanosized lactose gave more accurate dosing of drug.

### Example 6

Micronised lactose monohydrate (100 g) was dispersed in anhydrous ethanol (600 g) for 1 minute using a Silverson high shear mixer. This dispersion was added to the product reservoir of an Avestin Emulsiflex C50 homogeniser, and passed through the homogeniser at 10,000 psi and again at 21,000 psi. Part of the resulting dispersion (411g) was diluted with Anhydrous Ethanol to a weight of 645g, to achieve a ratio of liquid:solid::10:1 and to allow further processing. This composition was passed through the homogeniser at 20,000 psi. A batch was used for particle analysis (see below) and another batch for formulation testing (see below).

### Example 7

Micronised lactose monohydrate (59.7g) was dispersed in anhydrous ethanol (435.8 g) for 1 minute using a Silverson high shear mixer. This dispersion was added to the product reservoir of an Avestin Emulsiflex C50 homogenizer, and passed through the homogenizer at 10,000 psi and again at 20,000 psi. At the start of each passage, the dispersion was recirculated briefly to ensure that all product collected had passed through the homogenizer at the target pressure. The dispersion was then recirculated through the homogenizer continuously for 5 minutes at approximately 20,000 psi, and then sampled. Particle analysis was performed (see below).

### Example 8

Micronised lactose monohydrate (60.1g) was dispersed in anhydrous ethanol (438.7 g) for 1 minute using a Silverson high shear mixer. This dispersion was added to the product reservoir of an Avestin Emulsiflex C50 homogenizer, and passed through the homogenizer at 10,000 psi and again twice at 20,000 psi. At the start of each passage, the dispersion was recirculated briefly to ensure that all product collected had passed through the homogenizer at the target pressure. The dispersion was then sampled. Particle analysis was performed (see below).

### Particle Size Analysis

For analysis of a Lactose/Ethanol slurry, a (0.5 ml) sample of the slurry, which was shaken for at least one minute to ensure homogeneity, was added to a solution of 0.05% Lecithin in lso-octane (20 ml), and redispersed with mild ultrasonics for 1 minute.

For analysis of powdered Lactose (here double-micronised α-lactose monohydrate), a sample (500 mg) of the powder, which was previously shaken to ensure homogeneity, was added to a solution of 0.05% Lecithin in lso-octane (20 ml), and dispersed with mild ultrasonics for 1 minute.

The resulting suspension was introduced dropwise into the presentation cell (a Hydro 2000 SM small sample presentation cell) of a Malvern Mastersizer 2000™ laser diffraction particle sizer until the obscuration was in the working range (between 10 and 12 with a red laser), and left to circulate for 1 minute to allow complete mixing and thermal equilibrium to be established. Ten readings were taken at 20 second intervals to establish that the particle size was stable. The General Purpose analysis model, as described in the Malvern Instruments Operators Guide, was used with refractive indices 1.533 (lactose), 1.392 (isooctane) and absorbance 0.001 (lactose). The results are based on the average calculated results of 10 readings taken in succession. The procedure was performed twice.

### Results of Particle Size Analysis by Malvern Mastersizer 2000

| | Lactose, Double- micronised by fluid energy mill | Lactose, Example 6 | Lactose, Example 7 | Lactose, Example 8 |
|---|---|---|---|---|
| Units | Microns | Microns | Microns | Microns |
| d(v,0.1) | 2.103, 2.127 | 0.077, 0.077 | 0.086, 0.087 | 0.085, 0.084 |
| d(v,0.5) median | 3.385,3.590 | 0.193, 0.193 | 0.245, 0.250 | 0.250, 0.245 |
| d(v,0.9) | 5.721, 6.338 | 1.092, 1.100 | 1.201, 1.294 | 1.568, 1.645 |
| D[4,3] volume weighted mean | 3.691, 3.966 | 0.421, 0.429 | 0.459, 0.487 | 0.590, 0.611 |

| Units | Percent | Percent | Percent | Percent |
|---|---|---|---|---|
| Vol under 0.05 micron | 0.000, 0.000 | 1.73, 1.72 | 1.22, 1.18 | 1.29, 1.32 |
| Vol under 0.10 micron | 0.000, 0.000 | 19.55,19.50 | 14.48, 14.10 | 15.01, 15.34 |
| Vol under 0.20 micron | 0.000, 0.000 | 51.57, 51.49 | 41.73, 40.97 | 41.70, 42.37 |
| Vol under 0.50 micron | 0.000, 0.000 | 77.54, 77.50 | 72.10, 71.18 | 68.81, 68.57 |
| Vol under 1.0 micron | 0.000, 0.000 | 88.71, 88.62 | 86.42, 84.98 | 82.00,81.22 |
| Vol under 2.0 micron | 7.49, 7.20 | 97.16, 97.00 | 97.70, 96.83 | 93.64,92.98 |
| Vol under 5.0 micron | 82.69, 76.79 | 99.76, 99.66 | 100.00, 100.00 | 99.52, 99.38 |
| Vol under 10.0 micron | 100.00, 99.56 | 100.00, 100.00 | 100.00, 100.00 | 100.00, 100.00 |
| Vol under 20.0 micron | 100.00, 100.00 | 100.00, 100.00 | 100.00, 100.00 | 100.00, 100.00 |

### Formulation testing

Lactose dispersion from Example 6 was used to formulate inhalers with the following formulations:

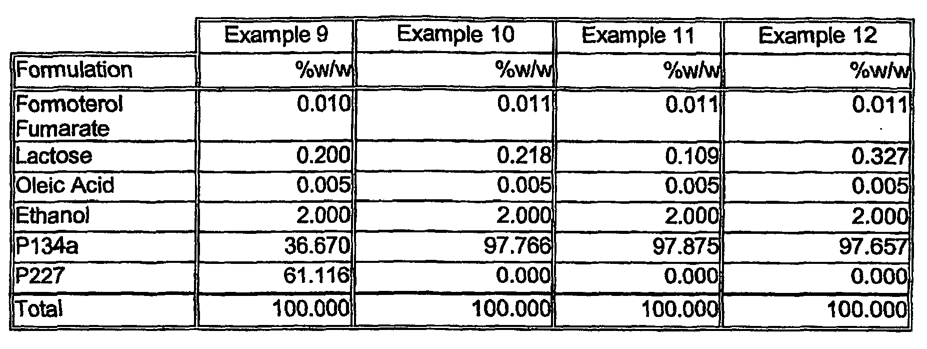

These were compared with equivalent formulations, in which the Lactose was prepared by double-micronising using a fluid energy mill, which are respectively designated formulations 9a -12a. (Mass median diameter, the average of the two values given above in the Table summarising the results of Particle Size Analysis, for Lactose (example 6) and double-micronised Lactose are 193 nm and 3.486 microns, respectively.)

It was found that the uniformity of delivered dose, as measured by relative standard deviation, was significantly better for the inhalers made using the Lactose prepared in example 6 than for those made from double-micronised Lactose.

Formulations, in which the ratio of Lactose to drug was 10:1 (Example 11) and 30:1 (Example 12) were compared with the formulation of example 10 (ratio 20:1). It was surprisingly found that the ratio of 20:1 gave superior uniformity of content than those with ratios 10:1 or 30:1.

| Example | Mean (n=15) | RSD |
|---|---|---|
| | (mcg/actuation) | (%) |
| 12 | 5.2 | 7.1 |
| 10 | 4.9 | 6.6 |
| 11 | 5.8 | 8.2 |
| 12a | 5.7 | 15.1 |
| 10a | 5.5 | 12.7 |
| 11a | 5.2 | 14.2 |

Furthermore, the loss of dose from the metering tank of the valve, which occurs when the product is allowed to stand for a prolonged period in the valve-up orientation, was compared for formulations 9 and 10, and the respective formulations 9a and 10a prepared from double-micronised Lactose. A reduced loss of dose was found when the inhalers were prepared using Lactose prepared in example 6 compared with those made from double-micronised Lactose.

### Example 13

Micronised lactose monohydrate (100 g) was dispersed in Anhydrous Ethanol (840 g) using a Silverson high shear mixer. This dispersion was added to the product reservoir of an Avestin Emulsiflex C50 homogenizer, and recirculated for 20 minutes at 10,000 psi. The dispersion was then passed out of the homogenizer at 20,000 psi.

### Example 14

Micronised lactose monohydrate (1063g) was dispersed in anhydrous ethanol (8929g) for 10 minutes using a Silverson high shear mixer. The mixture was added to a 20 litre stainless steel vessel and re-circulated through an Avestin Emulsiflex C160 high pressure homogeniser for 50 minutes at a pressure setting of 10,000psi. The resultant lactose/ethanol slurry was used to prepare the following lactose bulked suspension formulation of salbutamol sulphate:

| | Quantity (g) |
|---|---|
| Salbutamol sulphate (micronised) | 0.061 |
| Lactose/ethanol slurry | 0.571 (0.060 lactose/0.511 ethanol) |
| Ethanol | 1.94 |
| HFA 134a | 14.0 |

The formulation was prepared by weighing out the salbutamol sulphate into a clear plastic (PET) vial and then adding the lactose/ethanol slurry and ethanol. A non-metering valve was then crimped in place and the vial was sonicated in an ultrasonic water bath for one minute to disperse the solids. The HFA 134a was then injected into the vial to complete the formulation. A second vial (unbulked formulation) was prepared in which the lactose/ethanol slurry was omitted and replaced by a further quantity of ethanol (0.51 g).
The lactose bulked and the unbulked formulations of salbutamol sulphate were then visually compared and it was noted that the sedimentation rate was much slower for the bulked formulation. Floc heights were measured two minutes after shaking both vials. After two minutes, the floc height of the unbulked formulation filled 33% of the formulation volume, whereas the floc height of the bulked formulation filled 95% of the formulation volume. This observation indicates that by incorporation of sub-micron lactose into the formulation, a more dispersed and uniform suspension has been achieved.

### Example 15

DL-alanine (10g, used as supplied by Fisher Chemicals, Loughborough UK) was dispersed in anhydrous ethanol (200g) using a Silversen high shear mixer set at 10,000 RPM for 1 minute. The resultant dispersion was poured into the product vessel of a M110EH Microfluidizer. The Microfluidiser was fitted with an auxiliary process module of 100 micron channel diameter and a G10Z diamond interaction chamber of 87 micron channel diameter. The G10Z chamber was fitted downstream from the auxiliary process module. The dispersion was processed at 25,000 psi for 120 minutes.

A particle size analysis measurement was performed using a Malvern Mastersizer 2000™ laser diffraction particle sizer in an analogous manner to that described above for lactose, with the exception that a refractive index of 1.55 was employed for DL-alanine.

### Results of Particle Size Analysis by Malvern Mastersizer 2000

| | DL-Alanine |
|---|---|
| Units | Microns |
| d(v,0.1) | 0.077 |
| d(v,0.5) median | 0.190 |
| d(v,0.9) | 0.600 |
| D[4,3] volume weighted mean | 0.283 |

| Units | Percent |
|---|---|
| vol under 0.05 micron | 1.68 |
| vol under 0.10 micron | 19.13 |
| vol under 0.20 micron | 52.45 |
| vol under 0.50 micron | 86.56 |
| vol under 1.0 micron | 96.25 |
| vol under 2.0 micron | 99.98 |
| vol under 5.0 micron | 100.00 |
| vol under 10.0 micron | 100.00 |
| vol under 20.0 micron | 100.00 |

### Example 16

Sucrose (20g, used as supplied by British Sugar under the designation "Silk Sugar" (i.e. a fine grain icing sugar)) was dispersed in anhydrous ethanol (400g) using a Silverson high shear mixer set at 10,000 RPM for 1 minute. The resultant dispersion was poured into the product vessel of a M110EH Microfluidizer. The Microfluidizer was fitted with an auxiliary process module of 100 micron channel diameter and a G10Z diamond interaction chamber of 87 micron channel diameter. The G10Z chamber was fitted downstream from the auxiliary process module. The dispersion was processed at 25,000 psi for 60 minutes. A sample of the processed particles was examined by SEM, observed particle size was found in the range of 200 to 800 nanometers.

### Example 17

A formulation of salbutamol sulphate was prepared using DL-alanine/ethanol slurry from Example 15 as follows.

| | Quantity (g) |
|---|---|
| Salbutamol sulphate (micronised) | 0.061 |
| DL-alanine/ethanol slurry | 2.57 (0.12 DL-alanine / 2.45 ethanol) |
| P134a | 14.0 |

The formulation was prepared by weighing out the salbutamol sulphate into a clear plastic PET vial and then adding the DL-alanine/ethanol slurry. A non-metering valve was then crimped in place and the vial was sonicated in an ultrasonic water bath for 1 minute to disperse the solids. The P134a was then injected into the vial to complete the formulation. A second vial (unbulked formulation) was then prepared in which the DL-alanine/ethanol slurry was replaced by ethanol (2.45g).

The DL-alanine bulked and the unbulked formulations of salbutamol sulphate were visually compared and it was noted that the sedimentation rate was much slower for the bulked formulation. Floc heights were measured two minutes after shaking both vials. After two minutes, the floc height of the unbulked formulation filled 34% of the formulation volume, whereas floc height of the bulked formulation filled 99% of the formulation volume. This observation indicates that by incorporation of sub-micron DL-alanine into the formulation, a more uniform and dispersed suspension had been achieved.

### Example 18

A formulation of salbutamol sulphate was prepared using sucrose/ethanol slurry from Example 16 as follows:

| | Quantity (g) |
|---|---|
| Salbutamol sulphate (micronised) | 0.061 |
| sucrose/ethanol slurry | 2.57 (0.12 sucrose / 2.45 ethanol) |
| P134a | 14.0 |

The formulation was prepared by weighing out the salbutamol sulphate into a clear plastic PET vial and then adding the sucrose/ethanol slurry. A non-metering valve was then crimped in place and the vial was sonicated in an ultrasonic water bath for 1 minute to disperse the solids. The P134a was then injected into the vial to complete the formulation.

The sucrose bulked and the unbulked formulations of salbutamol sulphate were compared visually and it was noted that the sedimentation rate was much slower for the bulked formulation. Floc heights were measured two minutes after shaking both vials. After two minutes, the floc height of the unbulked formulation filled 34% of the formulation volume, whereas the floc height of the sucrose bulked formulation filled 80% of the formulation volume. This observation indicates that by incorporation of sub-micron sucrose into the formulation, a more uniform and dispersed suspension had been achieved.

## Claims

1. The use of a particulate bulking agent having a mass median diameter of less than one micron in pharmaceutical aerosol formulations comprising a suspension of drug particles in a propellant.

2. The use as claimed in Claim 1 in which the bulking agent is selected from ascorbic acid, saccharides, polysaccharides, amino acids, organic and inorganic salts, urea and propyliodone.

3. The use as claimed in Claim 2 in which the bulking agent is selected from lactose, DL-alanine, glucose, D-galactose, D(+)trehalose dihydrate, sucrose, maltose, D(+)raffinose pentahydrate, sodium saccharin, starches, modified celluloses, dextrins, dextrans, glycine, sodium chloride, calcium carbonate, sodium tartrate and calcium lactate.

4. The use as claimed in any preceding claim in which the bulking agent is lactose.

5. The use as claimed in any preceding claim in which the bulking agent has a mass median diameter of not more than 300nm.

6. The use as claimed in any preceding claim in which the bulking agent has a mass median diameter of not more than 250 nm.

7. The use as claimed in any preceding claim in which the weight ratio of drug : bulking agent is in the range 1:0.1 to 1:100.

8. The use as claimed in Claim 7 in which the weight ratio of drug : bulking agent is in the range 1:5 to 1:40.

9. The use as claimed in any preceding claim in which the drug has a mass median diameter equal to or greater than 1 micron.

10. The use as claimed in any one of Claims 1 to 8 in which the drug has a mass median diameter of less than one micron.

11. The use as claimed in any preceding claim in which the bulking agent is added to the formulation in the form of a slurry or thick paste.

12. The use as claimed in Claim 11 in which the bulking agent is added to the formulation in the form of a slurry or thick paste in ethanol.

13. The use as claimed in any preceding claim in which the drug is selected from Formoterol, Salmeterol, Fluticasone Propionate, Procaterol and Ipratropium and salts thereof.

14. The use as claimed in Claim 13 in which the drug is Formoterol.

15. The use as claimed in any preceding claim in which the propellant is a hydrofluoroalkane.

16. The use as claimed in Claim 15 in which the propellant is selected from HFA 134a, HFA 227 and mixtures thereof.

17. A pharmaceutical aerosol formulation comprising particles of drug dispersed in a propellant and a bulking agent having a mass median diameter of less than one micron.

18. A pharmaceutical aerosol formulation as claimed in Claim 17 in which the bulking agent is selected from ascorbic acid, saccharides, polysaccharides, amino acids, organic and inorganic salts, urea and propyliodone.

19. A pharmaceutical aerosol formulation as claimed in Claim 18 in which the bulking agent is selected from lactose, DL-alanine, glucose, D-galactose, D(+)trehalose dihydrate, sucrose, maltose, D(+)raffinose pentahydrate, sodium saccharin, starches, modified celluloses, dextrins, dextrans, glycine, sodium chloride, calcium carbonate, sodium tartrate and calcium lactate.

20. A pharmaceutical aerosol formulation as claimed in any one of Claims 17 to 19 in which the bulking agent is lactose.

21. A pharmaceutical aerosol formulation as claimed in any one of Claims 17 to 20 in which the bulking agent has a mass median diameter of not more than 300 nm.

22. A pharmaceutical aerosol formulation as claimed in any one claims 17 to 21 in which the bulking agent has a mass median diameter of not more than 250 nm.

23. A pharmaceutical aerosol formulation as claimed in any one of Claims 17 to 22 in which the weight ratio of drug : bulking agent is in the range 1:0.1 to 1:100.

24. A pharmaceutical aerosol formulation as claimed in Claim 23 in which the weight ratio of drug : bulking agent is in the range 1:5 to 1:40.

25. A pharmaceutical aerosol formulation as claimed in any one of Claims 17 to 24 in which the drug has a mass median diameter equal to or greater than 1 micron.

26. A pharmaceutical aerosol formulation as claimed in any one of Claims 17 to 25 in which the drug has a mass median diameter of less than one micron.

27. A pharmaceutical aerosol formulation as claimed in any one of Claims 17 to 26 in which the drug is selected from Formoterol, Salmeterol, Fluticasone Propionate, Procaterol and Ipratropium and salts thereof.

28. A pharmaceutical aerosol formulation as claimed in Claim 27 in which the drug is Formoterol.

29. A pharmaceutical aerosol formulation as claimed in claim 28 in which the weight ratio of drug : bulking agent is about 1:20.

30. A pharmaceutical aerosol formulation as claimed in any one of Claims 17 to 29 in which the propellant is a hydrofluoroalkane.

31. A pharmaceutical aerosol formulation as claimed in Claim 30 in which the propellant is selected from HFA 134a, HFA 227 and mixtures thereof.

32. A pharmaceutical aerosol formulation as claimed in any one of Claims 17 to 31 which additionally comprises a surfactant.

33. A pharmaceutical aerosol formulation as claimed in Claim 32 in which the surfactant is selected from sorbitan trioleate, oleic acid and oligolactic acid derivatives.

34. A pharmaceutical aerosol formulation as claimed in any one of Claims 17 to 33 which additionally comprises ethanol.

35. A pharmaceutical aerosol formulation as claimed in Claim 34 in which the ethanol comprises from 0.1 to 5% by weight of the formulation.

36. A pharmaceutical aerosol formulation as claimed in Claim 35 in which the ethanol comprises from about 0.5 to about 3% by weight of the formulation.

37. A pharmaceutical aerosol formulation as claimed in Claim 36 in which the ethanol comprises from about 1 to about 2% by weight of the formulation.

38. A process for the preparation of an aerosol formulation comprising a suspension of particles in propellant which process comprises forming a slurry of bulking agent with a component of the aerosol formulation, subjecting the slurry to high pressure homogenization to reduce the particle size of the bulking agent, and thereafter mixing the resulting slurry with other components of the aerosol formulation.

39. A process as claimed in Claim 38 in which the slurry of bulking agent has a liquid : solid weight ratio of from 5:1 to 100:1.

40. A process as claimed in Claim 39 in which the slurry of bulking agent has a liquid : solid weight ratio of from 5:1 to 20:1.

41. A process as claimed in claim 40 in which the slurry of bulking agent has a liquid : solid weight ratio of about 10:1.

42. A process as claimed in any one of Claims 38 to 41 comprising the step of adjusting the liquid : solid ratio of the slurry after homogenisation before mixing with other components of the aerosol formulation.

43. A process as claimed in any one of Claims 38 to 42 in which the bulking agent is reduced to particles having a mass median diameter of not more than one micron.

## Revendications

1. Utilisation d'un agent de charge en particules d'un diamètre médian en masse inférieur à un micromètre dans des formulations pharmaceutiques en aérosol qui comprennent une suspension de particules de médicament dans un propulseur.

2. Utilisation selon la revendication 1, dans laquelle l'agent de charge est sélectionné parmi l'acide ascorbique, les saccharides, les polysaccharides, les acides aminés, les sels organiques et minéraux, l'urée et la propyliodone.

3. Utilisation selon la revendication 2, dans laquelle l'agent de charge est sélectionné parmi le lactose, la DL-alanine, le glucose, le D-galactose, le D(+)tréhalose dihydraté, le sucrose, le maltose, le D(+)raffinose pentahydraté, la saccharine de sodium, les amidons, les celluloses modifiées, les dextrines, les dextranes, la glycine, le chlorure de sodium, le carbonate de calcium, le tartrate de sodium et le lactate de calcium.

4. Utilisation selon l'une quelconque des revendications précédentes, dans laquelle l'agent de charge est le lactose.

5. Utilisation selon l'une quelconque des revendications précédentes, dans laquelle l'agent de charge a un diamètre médian en masse non supérieur à 300 nm.

6. Utilisation selon l'une quelconque des revendications précédentes, dans laquelle l'agent de charge a un diamètre médian en masse non supérieur à 250 nm.

7. Utilisation selon l'une quelconque des revendications précédentes, dans laquelle le rapport pondéral entre le médicament et l'agent de charge est compris dans la plage de 1 : 0,1 à 1 : 100.

8. Utilisation selon la revendication 7, dans laquelle le rapport pondéral entre le médicament et l'agent de charge est compris dans la plage de 1 : 5 à 1 : 40.

9. Utilisation selon l'une quelconque des revendications précédentes, dans laquelle le médicament a un diamètre médian en masse égal ou supérieur à 1 micromètre.

10. Utilisation selon l'une quelconque des revendications 1 à 8, dans laquelle le médicament a un diamètre médian en masse inférieur à 1 micromètre.

11. Utilisation selon l'une quelconque des revendications précédentes, dans laquelle l'agent de charge est ajouté à la formulation sous la forme d'une boue ou d'une pâte épaisse.

12. Utilisation selon la revendication 11, dans laquelle l'agent de charge est ajouté à la formulation sous la forme d'une boue ou d'une pâte épaisse dans de l'éthanol.

13. Utilisation selon l'une quelconque des revendications précédentes, dans laquelle le médicament est sélectionné parmi le formotérol, le salmétérol, le propionate de fluticasone, le procatérol et l'ipratropium, ainsi que leurs sels.

14. Utilisation selon la revendication 13, dans laquelle le médicament est le formotérol.

15. Utilisation selon l'une quelconque des revendications précédentes, dans laquelle le propulseur est un hydrofluoroalcane.

16. Utilisation selon la revendication 15, dans laquelle le propulseur est sélectionné parmi l'HFA 134a, l'HFA 227 et leurs mélanges.

17. Formulation pharmaceutique en aérosol qui comprend des particules de médicament dispersées dans un propulseur et un agent de charge dont le diamètre médian en masse est inférieur à un micromètre.

18. Formulation pharmaceutique en aérosol selon la revendication 17, dans laquelle l'agent de charge est sélectionné parmi l'acide ascorbique, les saccharides, les polysaccharides, les acides aminés, les sels organiques et minéraux, l'urée et la propyliodone.

19. Formulation pharmaceutique en aérosol selon la revendication 18, dans laquelle l'agent de charge est sélectionné parmi le lactose, la DL-alanine, le glucose, le D-galactose, le D(+)tréhalose dihydraté, le sucrose, le maltose, le D(+)raffinose pentahydraté, la saccharine de sodium, les amidons, les celluloses modifiées, les dextrines, les dextranes, la glycine, le chlorure de sodium, le carbonate de calcium, le tartrate de sodium et le lactate de calcium.

20. Formulation pharmaceutique en aérosol selon l'une quelconque des revendications 17 à 19, dans laquelle l'agent de charge est le lactose.

21. Formulation pharmaceutique en aérosol selon l'une quelconque des revendications 17 à 20, dans laquelle l'agent de charge a un diamètre médian en masse non supérieur à 300 nm.

22. Formulation pharmaceutique en aérosol selon l'une quelconque des revendications 17 à 21, dans laquelle l'agent de charge a un diamètre médian en masse non supérieur à 250 nm.

23. Formulation pharmaceutique en aérosol selon l'une quelconque des revendications 17 à 22, dans laquelle le rapport pondéral entre le médicament et l'agent de charge est compris dans la plage de 1 : 0,1 à 1 : 100.

24. Formulation pharmaceutique en aérosol selon la revendication 23, dans laquelle le rapport pondéral entre le médicament et l'agent de charge est compris dans la plage de 1 : 5 à 1 : 40.

25. Formulation pharmaceutique en aérosol selon l'une quelconque des revendications 17 à 24, dans laquelle le médicament a un diamètre médian en masse égal ou supérieur à 1 micromètre.

26. Formulation pharmaceutique en aérosol selon l'une quelconque des revendications 17 à 25, dans laquelle le médicament a un diamètre médian en masse inférieur à 1 micromètre.

27. Formulation pharmaceutique en aérosol selon l'une quelconque des revendications 17 à 26, dans laquelle le médicament est sélectionné parmi le formotérol, le salmétérol, le propionate de fluticasone, le procatérol et l'ipratropium, ainsi que leurs sels.

28. Formulation pharmaceutique en aérosol selon la revendication 27, dans laquelle le médicament est le formotérol.

29. Formulation pharmaceutique en aérosol selon la revendication 28, dans laquelle le rapport pondéral entre le médicament et l'agent de charge est d'environ 1 : 20.

30. Formulation pharmaceutique en aérosol selon l'une quelconque des revendications 17 à 29, dans laquelle le propulseur est un hydrofluoroalcane.

31. Formulation pharmaceutique en aérosol selon la revendication 30, dans laquelle le propulseur est sélectionné parmi l'HFA 134a, l'HFA 227 et leurs mélanges.

32. Formulation pharmaceutique en aérosol selon l'une quelconque des revendications 17 à 31, qui comprend en supplément un agent tensioactif.

33. Formulation pharmaceutique en aérosol selon la revendication 32, dans laquelle l'agent tensioactif est sélectionné parmi le trioléate de sorbitane, l'acide oléique et les dérivés d'acide oligolactique.

34. Formulation pharmaceutique en aérosol selon l'une quelconque des revendications 17 à 33, qui comprend de plus de l'éthanol.

35. Formulation pharmaceutique en aérosol selon la revendication 34, dans laquelle l'éthanol constitue de 0,1 à 5 % en poids de la formulation.

36. Formulation pharmaceutique en aérosol selon la revendication 35, dans laquelle l'éthanol constitue d'environ 0,5 à environ 3 % en poids de la formulation.

37. Formulation pharmaceutique en aérosol selon la revendication 36, dans laquelle l'éthanol constitue d'environ 1 à environ 2 % en poids de la formulation.

38. Procédé de préparation d'une formulation en aérosol qui comprend une suspension de particules dans un propulseur, lequel procédé comporte les étapes qui consistent à former une boue d'agent de charge avec un composant de la formulation en aérosol, à faire subir à la boue une homogénéisation sous haute pression pour réduire la taille des particules de l'agent de charge et ensuite à mélanger la boue ainsi obtenue avec les autres composants de la formulation en aérosol.

39. Procédé selon la revendication 38, dans lequel la boue d'agent de charge présente un rapport pondéral liquide : solide de 5 : 1 à 100 : 1.

40. Procédé selon la revendication 39, dans lequel la boue d'agent de charge présente un rapport pondéral liquide : solide de 5 : 1 à 20 : 1.

41. Procédé selon la revendication 40, dans lequel la boue d'agent de charge présente un rapport pondéral liquide : solide d'environ 10 : 1.

42. Procédé selon l'une quelconque des revendications 38 à 41, qui comporte l'étape qui consiste à ajuster le rapport liquide : solide de la boue après homogénéisation et avant mélange avec d'autres composants de la formulation en aérosol.

43. Procédé selon l'une quelconque des revendications 38 à 42, dans lequel l'agent de charge est réduit en particules dont le diamètre médian en masse n'est pas supérieur à un micromètre.

## Patentansprüche

1. Verwendung eines teilchenförmigen Füllmittels mit einem massengemittelten Durchmesser von weniger als einem Mikrometer in pharmazeutischen Aerosolformulierungen, die eine Suspension von Arzneistoffpartikeln in einem Treibmittel umfassen.

2. Verwendung nach Anspruch 1, wobei das Füllmittel aus Ascorbinsäure, Sacchariden, Polysacchariden, Aminosäuren, organischen und anorganischen Salzen, Harnstoff und Propyliodon ausgewählt ist.

3. Verwendung nach Anspruch 2, wobei das Füllmittel aus Lactose, DL-Alanin, Glucose, D-Galactose, D(+)-Trehalosedihydrat, Saccharose, Maltose, D(+)-Raffinosepentahydrat, Saccharin-Natrium, Stärken, modifizierten Cellulosen, Dextrinen, Dextranen, Glycin, Natriumchlorid, Calciumcarbonat, Natriumtartrat und Calciumlactat ausgewählt ist.

4. Verwendung nach einem der vorhergehenden Ansprüche, wobei das Füllmittel Lactose ist.

5. Verwendung nach einem der vorhergehenden Ansprüche, wobei das Füllmittel einen massengemittelten Durchmesser von nicht mehr als 300 nm hat.

6. Verwendung nach einem der vorhergehenden Ansprüche, wobei das Füllmittel einen massengemittelten Durchmesser von nicht mehr als 250 nm hat.

7. Verwendung nach einem der vorhergehenden Ansprüche, wobei das Gewichtsverhältnis von Arzneistoff:Füllmittel im Bereich von 1:0,1 bis 1:100 liegt.

8. Verwendung nach Anspruch 7, wobei das Gewichtsverhältnis von Arzneistoff:Füllmittel im Bereich von 1:5 bis 1:40 liegt.

9. Verwendung nach einem der vorhergehenden Ansprüche, wobei der Arzneistoff einen massengemittelten Durchmesser gleich oder größer als 1 Mikrometer hat.

10. Verwendung nach einem der Ansprüche 1 bis 8, wobei der Arzneistoff einen massengemittelten Durchmesser von weniger als einem Mikrometer hat.

11. Verwendung nach einem der vorhergehenden Ansprüche, wobei das Füllmittel der Formulierung in Form einer Aufschlämmung oder dicken Paste zugegeben wird.

12. Verwendung nach Anspruch 11, wobei das Füllmittel der Formulierung in Form einer Aufschlämmung oder dicken Paste in Ethanol zugegeben wird.

13. Verwendung nach einem der vorhergehenden Ansprüche, wobei der Arzneistoff aus Formoterol, Salmeterol, Fluticasonpropionat, Procaterol und Ipratropium und Salzen davon ausgewählt ist.

14. Verwendung nach Anspruch 13, wobei der Arzneistoff Formoterol ist.

15. Verwendung nach einem der vorhergehenden Ansprüche, wobei das Treibmittel ein Hydrofluoralkan ist.

16. Verwendung nach Anspruch 15, wobei das Treibmittel aus HFA 134a, HFA 227 und Mischungen davon ausgewählt ist.

17. Pharmazeutische Aerosolformulierung, die in einem Treibmittel dispergierte Arzneistoffpartikel und ein Füllmittel mit einem massengemittelten Durchmesser von weniger als einem Mikrometer umfasst.

18. Pharmazeutische Aerosolformulierung nach Anspruch 17, wobei das Füllmittel aus Ascorbinsäure, Sacchariden, Polysacchariden, Aminosäuren, organischen und anorganischen Salzen, Harnstoff und Propyliodon ausgewählt ist.

19. Pharmazeutische Aerosolformulierung nach Anspruch 18, wobei das Füllmittel aus Lactose, DL-Alanin, Glucose, D-Galactose, D(+)-Trehalosedihydrat, Saccharose, Maltose, D(+)-Raffinosepentahydrat, Saccharin-Natrium, Stärken, modifizierten Zellulosen, Dextrinen, Dextranen, Glycin, Natriumchlorid, Calciumcarbonat, Natriumtartrat und Calciumlactat ausgewählt ist.

20. Pharmazeutische Aerosolformulierung nach einem der Ansprüche 17 bis 19, wobei das Füllmittel Lactose ist.

21. Pharmazeutische Aerosolformulierung nach einem der Ansprüche 17 bis 20, wobei das Füllmittel einen massengemittelten Durchmesser von nicht mehr als 300 nm hat.

22. Pharmazeutische Aerosolformulierung nach einem der Ansprüche 17 bis 21, wobei das Füllmittel einen massengemittelten Durchmesser von nicht mehr als 250 nm hat.

23. Pharmazeutische Aerosolformulierung nach einem der Ansprüche 17 bis 22, wobei das Gewichtsverhältnis von Arzneistoff:Füllmittel im Bereich von 1:0,1 bis 1:100 liegt.

24. Pharmazeutische Aerosolformulierung nach Anspruch 23, wobei das Gewichtsverhältnis von Arzneistoff:Füllmittel im Bereich von 1:5 bis 1:40 liegt.

25. Pharmazeutische Aerosolformulierung nach einem der Ansprüche 17 bis 24, wobei das Arzneimittel einen massengemittelten Durchmesser gleich oder größer als 1 Mikrometer hat.

26. Pharmazeutische Aerosolformulierung nach einem der Ansprüche 17 bis 25, wobei der Arzneistoff einen massengemittelten Durchmesser von weniger als einem Mikrometer hat.

27. Pharmazeutische Aerosolformulierung nach einem der Ansprüche 17 bis 26, wobei der Arzneistoff aus Formoterol, Salmeterol, Fluticasonpropionat, Procaterol und Ipratropium und Salzen davon ausgewählt ist.

28. Pharmazeutische Aerosolformulierung nach Anspruch 27, wobei der Arzneistoff Formoterol ist.

29. Pharmazeutische Aerosolformulierung nach Anspruch 28, wobei das Gewichtsverhältnis von Arzneistoff: Füllmittel etwa 1:20 beträgt.

30. Pharmazeutische Aerosolformulierung nach einem der Ansprüche 17 bis 29, wobei das Treibmittel ein Hydrofluoralkan ist.

31. Pharmazeutische Aerosolformulierung nach Anspruch 30, wobei das Treibmittel aus HFA 134a, HFA 227 und Mischungen davon ausgewählt ist.

32. Pharmazeutische Aerosolformulierung nach einem der Ansprüche 17 bis 31, die zusätzlich ein oberflächenaktives Mittel umfasst.

33. Pharmazeutische Aerosolformulierung nach Anspruch 32, wobei das oberflächenaktive Mittel aus Sorbitantrioleat, Ölsäure und Oligomilchsäurederivaten ausgewählt ist.

34. Pharmazeutische Aerosolformulierung nach einem der Ansprüche 17 bis 33, die zusätzlich Ethanol umfasst.

35. Pharmazeutische Aerosolformulierung nach Anspruch 34, wobei das Ethanol 0,1 bis 5 Gew.-% der Formulierung ausmacht.

36. Pharmazeutische Aerosolformulierung nach Anspruch 35, wobei das Ethanol etwa 0,5 bis etwa 3 Gew.-% der Formulierung ausmacht.

37. Pharmazeutische Aerosolformulierung nach Anspruch 36, wobei das Ethanol etwa 1 bis etwa 2 Gew.-% der Formulierung ausmacht.

38. Verfahren zur Herstellung einer Aerosolformulierung, die eine Suspension von Partikeln in Treibmittel umfasst, wobei in dem Verfahren eine Aufschlämmung von Füllmittel mit einer Komponente der Aerosolformulierung gebildet wird, die Aufschlämmung Hochdruckhomogenisierung zur Reduktion der Partikelgröße des Füllmittels unterzogen wird und anschließend die resultierende Aufschlämmung mit anderen Komponenten der Aerosolformulierung gemischt wird.

39. Verfahren nach Anspruch 38, wobei die Aufschlämmung des Füllmittels ein Gewichtsverhältnis von Flüssigkeit:Feststoff von 5:1 bis 100:1 hat.

40. Verfahren nach Anspruch 39, wobei die Aufschlämmung des Füllmittels ein Gewichtsverhältnis von Flüssigkeit:Feststoff von 5:1 bis 20:1 hat.

41. Verfahren nach Anspruch 40, wobei die Aufschlämmung des Füllmittels ein Gewichtsverhältnis von Flüssigkeit:Feststoff von etwa 10:1 hat.

42. Verfahren nach einem der Ansprüche 38 bis 41, das die Stufe des Einstellens des Verhältnisses von Flüssigkeit:Feststoff in der Aufschlämmung nach der Homogenisierung umfasst, bevor mit anderen Komponenten der Aerosolformulierung gemischt wird.

43. Verfahren nach einem der Ansprüche 38 bis 42, wobei das Füllmittel zu Partikeln mit einem massengemittelten Durchmesser von nicht mehr als einem Mikrometer reduziert wird.
